# EUROPÄISCHE PATENTANMELDUNG

(11) **EP 2 505 080 A1**
(43) Veröffentlichungstag der Anmeldung: **03.10.2012**
(21) Anmeldenummer: 11178028.4
(22) Anmeldetag: 18.08.2011
(51) Int. Cl.: A23L 1/30, A23L 2/52, A61K 36/28, A61K 36/899, A61K 36/48

(54) **Gesundheitsfördernde Getränkezusammensetzung**

(71) Anmelder: Pegel, Sybille, 22043 Hamburg (DE); Kaesemann, Vera, 20355 Hamburg (DE)
(72) Erfinder: Pegel, Sybille, 22043 Hamburg (DE); Kaesemann, Vera, 20355 Hamburg (DE)
(74) Vertreter: UEXKÜLL & STOLBERG

(57) **Zusammenfassung**

Die Erfindung betrifft eine gesundheitsfördernde Getränkezusammensetzung, die zerkleinerte Pflanzenteile von Pflanzen ausgewählt aus mindestens zwei der Gruppen Hülsenfrüchtler, Korbblütler und Süßgräser enthält. Die Erfindung betrifft auch ein Verfahren zur Herstellung der Getränkezusammensetzung sowie deren Verwendung.

## Beschreibung

Die Erfindung betrifft eine gesundheitsfördernde Getränkezusammensetzung, die zerkleinerte Pflanzenteile von unterschiedlichen Pflanzen enthält. Die Erfindung betrifft auch ein Verfahren zur Herstellung der Getränkezusammensetzung sowie deren Verwendung.

Tryptophan ist eine proteinogene aromatische a-Aminosäure. Es handelt sich dabei um eine essentielle Aminosäure, die nicht vom menschlichen Körper synthetisiert werden kann, sondern in Form von tryptophanhaltigen Proteinen mit der Nahrung zugeführt werden muss.

Tryptophan ist ein metabolischer Vorläufer des Neurotransmitters Serotonin. Serotonin hat im Zentralnervensystem unter anderem wesentlichen Einfluss auf Stimmung und Verhalten. So kann ein Serotoninmangel im Gehirn zur Entstehung affektiver Störungen wie beispielsweise Depressionen beitragen. Da Serotonin selbst nicht in der Lage ist, die Blut-Hirn-Schranke zu passieren, kann ein Serotoninmangel im Gehirn nicht durch Gabe von Serotonin behoben werden. Das im Zentralnervensystem vorhandene Serotonin wird über die Zwischenstufe 5-Hydroxytryptophan aus Tryptophan gebildet. Voraussetzung für die erforderliche Bildung von Serotonin ist daher unter anderem eine ausreichende Verfügbarkeit von Tryptophan im Gehirn.

Es ist versucht worden, durch Erhöhung der Tryptophanzufuhr die Bildung von Serotonin im Zentralnervensystem zu fördern und so mit einem Mangel an Serotonin zusammenhängende Symptome zu lindern. So ist etwa die Verwendung von freiem Tryptophan als Arzneimittel zur Behandlung von Schlafstörungen bekannt. Es hat sich jedoch herausgestellt, dass eine Erhöhung der Tryptophanzufuhr in den meisten Fällen nicht ausreicht, um die Bildung von Serotonin im Gehirn entscheidend zu anzuregen, da der Transport von Tryptophan über die Blut-Hirn-Schranke von zahlreichen Faktoren abhängt.

Unter dem Namen FROHNATUR^{®} wird eine Mischung von Tryptophan mit Glucose, Mineralstoffen und Vitaminen angeboten, die eine stimmungsverbessernde Wirkung haben soll. Die enthaltene Glucose und die durch diese bewirkte Ausschüttung von Insulin sollen sich günstig auf die Aufnahme von Tryptophan in das Gehirn auswirken.

Unter dem Namen Inka Gold werden Kapseln angeboten, die eine Mischung von Pulvern der eng verwandten Pseudogetreide Quinoa und Amaranth enthalten. Die Kapseln werden mit etwas Flüssigkeit eingenommen und sollen das Wohlbefinden steigern.

WO 01/89319 A2 beschreibt Zusammensetzungen, die ein zumindest teilweise entfettetes, tryptophanhaltiges Mehl einer pflanzlichen Quelle wie vorzugsweise Kürbiskernen und eine Kohlenhydratquelle in einer Menge enthalten, die geeignet ist, den Transport von Tryptophan über die Blut-Hirn-Schranke zu fördern. Vorzugsweise enthalten die Zusammensetzungen Kohlenhydrate mit hohem glykämischem Index, insbesondere Glucose, wodurch die Aufnahme von Tryptophan über die Blut-Hirn-Schranke erleichtert werden soll. Die Zusammensetzungen sollen unter anderem das Einschlafen fördern.

Es hat sich jedoch gezeigt, dass die bekannten tryptophanhaltigen Zusammensetzungen nicht in der Lage sind, eine optimale Verfügbarkeit von Tryptophan zur Aufnahme in das Gehirn bereitzustellen. Zudem sind Zusammensetzungen, die freies Tryptophan und/oder freie Zusatzstoffe wie Kohlenhydrate, Fette oder Proteine enthalten, für viele Anwendungsbereiche, insbesondere für Produkte auf Basis natürlicher Inhaltsstoffe, nicht akzeptabel.

Es besteht daher ein Bedarf an einer tryptophanhaltigen Zusammensetzung, die die Nachteile des Standes der Technik überwindet und insbesondere auf Basis natürlicher Inhaltsstoffe eine effiziente Aufnahme von Tryptophan in das Gehirn und eine Anregung des Serotoninstoffwechsels ohne die Notwendigkeit des Zusatzes von freiem Tryptophan oder anderen Zusatzstoffen ermöglicht.

Diese Aufgabe wird durch die erfindungsgemäße Getränkezusammensetzung gelöst.

In einem ersten Aspekt betrifft die Erfindung eine gesundheitsfördernde Getränkezusammensetzung, die zerkleinerte Pflanzenteile von Pflanzen ausgewählt aus den Gruppen Hülsenfrüchtler, Korbblütler und Süßgräser enthält. Vorzugsweise enthält die Getränkezusammensetzung zerkleinerte Pflanzenteile von Pflanzen ausgewählt aus mindestens zwei der Gruppen Hülsenfrüchtler, Korbblütler und Süßgräser.

Der Begriff "Pflanzenteil" bezeichnet hier allgemein jeglichen morphologischen Teil einer Pflanze einschließlich der Frucht. Dabei handelt es sich grundsätzlich um Pflanzenteile, die für den Menschen nicht giftig sind. Bevorzugt sind pflanzliche Samen, Knollen und Körner.

Der Begriff "zerkleinert" bezeichnet einen Pflanzenteil, der mechanisch, beispielsweise durch einen Mahlvorgang, zerkleinert ist. Bevorzugt liegen die Pflanzenteile in gemahlener Form, beispielsweise als Mehl, Grieß, Schrot oder Kleie, und insbesondere als Mehl vor.

Soweit nicht anders angegeben beziehen sich alle Prozentangaben auf das Gesamtgewicht der Getränkezusammensetzung.

Es wurde überraschend gefunden, dass insbesondere durch eine Kombination von zerkleinerten Pflanzenteilen aus mindestens zwei ganz unterschiedlichen pflanzlichen Quellen und deren Formulierung als Getränk proteingebundenes Tryptophan in einer Form zur Verfügung gestellt wird, die auf Basis natürlicher Inhaltsstoffe und ohne die Notwendigkeit der Verwendung von Zusätzen die Aufnahme von Tryptophan in das Gehirn und seine Umwandlung zu Serotonin besonders begünstigt.

Die erfindungsgemäße Getränkezusammensetzung macht dabei offenbar neben Tryptophan weitere Nährstoffe und Cofaktoren in einer Kombination verfügbar, durch welche die am Transport von Tryptophan über die Blut-Hirn-Schranke und die Bildung von Serotonin im Gehirn beteiligten physiologischen Systeme positiv beeinflusst werden. Relevante Cofaktoren sind insbesondere die Vitamine B1, B3, B6, B12 und C, Folsäure sowie Zink und Calcium. Tryptophan passiert die Blut-Hirn-Schranke über ein aktives Transportsystem, um das es üblicherweise mit verschiedenen anderen Aminosäuren konkurriert. Zudem ist Tryptophan im Plasma zu einem großen Teil an Serumalbumin gebunden und steht in dieser Form für die Passage der Blut-Hirn-Schranke nicht zur Verfügung. Die Verfügbarkeit von freiem Tryptophan im Gehirn hängt daher neben der Menge, in der Tryptophan dem Körper beispielsweise in proteingebundener Form zugeführt wird, von verschiedenen weiteren Bedingungen ab. Diese Bedingungen werden durch die erfindungsgemäße Getränkezusammensetzung zugunsten einer effektiven Erhöhung des Serotoninspiegels im Gehirn beeinflusst.

Bevorzugt enthält die erfindungsgemäße Getränkezusammensetzung
(a) zerkleinerte Pflanzenteile, insbesondere gemahlene Samen, mindestens einer Pflanze aus der Gruppe der Hülsenfrüchtler;
(b) zerkleinerte Pflanzenteile, insbesondere gemahlene Knollen, mindestens einer Pflanze aus der Gruppe der Korbblütler; und
(c) zerkleinerte Pflanzenteile, insbesondere gemahlene Körner, mindestens einer Pflanze aus der Gruppe der Süßgräser.

Geeignete Hülsenfrüchtler sind beispielsweise Schmetterlingsblütler. Hülsenfrüchtler werden erfindungsgemäß insbesondere in Form zerkleinerter Samen und vorzugsweise als Mehl verwendet. Dabei sind Hülsenfrüchtler bevorzugt, deren Samen bezogen auf die Trockenmasse mindestens 30 Gew.-% und insbesondere mindestens 35 Gew.-% Proteine enthalten und einen Tryptophan-Anteil von mindestens 0,5 g/100 g Protein und insbesondere mindestens 1 g/100 g Protein aufweisen. Weiterhin ist es bevorzugt, dass die Samen der Hülsenfrüchtler bezogen auf die Trockenmasse weniger als 10 Gew.-% und insbesondere weniger als 7,5 Gew.-% Kohlenhydrate und/oder weniger als 10 Gew.-% und insbesondere weniger als 7,5 Gew.-% Fett enthalten. Die Samen der Hülsenfrüchtler weisen vorzugsweise mindestens 1 µg und insbesondere mindestens 2 µg Vitamin B12/100 g auf. Ein besonders bevorzugter Hülsenfrüchtler ist Lupine, vorzugsweise Süßlupine, insbesondere in Form von Lupinenmehl. Typischerweise enthält die Getränkezusammensetzung 0,1 bis 20 Gew.-%, insbesondere 0,5 bis 10 Gew.-%, bevorzugt 1 bis 5 Gew.-%, besonders bevorzugt 2 bis 4 Gew.-% und am meisten bevorzugt 2,5 bis 3, 5 Gew.-% zerkleinerte Pflanzenteile mindestens einer Pflanze aus der Gruppe der Hülsenfrüchtler.

Geeignete Korbblütler sind beispielsweise Pflanzen aus der Gattung der Sonnenblumen. Korbblütler werden erfindungsgemäß insbesondere in Form zerkleinerter Knollen und vorzugsweise als Mehl verwendet. Dabei sind Korbblütler bevorzugt, deren Knollen bezogen auf die Trockenmasse mindestens 5 Gew.-% und insbesondere mindestens 10 Gew.-% Protein enthalten und dabei insbesondere einen Tryptophan-Anteil von mindestens 0,25 g/100 g Protein und insbesondere mindestens 0,5 g/100 g Protein aufweisen. Vorzugsweise enthalten die Knollen bezogen auf die Trockenmasse weniger als 5 Gew.-% und insbesondere weniger als 2,5 Gew.-% Fett und/oder mindestens 50 Gew.-% und insbesondere mindestens 60 Gew.-% Kohlenhydrate. Insbesondere sind solche Korbblütler bevorzugt, deren Knollen bezogen auf die Trockenmasse mindestens 20 Gew.-%, insbesondere mindestens 40 Gew.-% und am meisten bevorzugt mindestens 50 Gew.-% prebiotische Kohlenhydrate wie Inulin enthalten. Weiterhin ist es bevorzugt, dass die Knollen bezogen auf die Trockenmasse mindestens 0,5 Gew.-%, insbesondere mindestens 1 Gew.-% und besonders bevorzugt mindestens 2 Gew.-% Kalium enthalten. Es hat sich gezeigt, dass Kalium eine wichtige Rolle beim Transport von Serotonin im Liquor spielt. Ein besonders bevorzugter Korbblütler ist Topinambur, insbesondere in Form von Topinamburknollenmehl. Typischerweise enthält die Getränkezusammensetzung 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% zerkleinerte Pflanzenteile mindestens einer Pflanze aus der Gruppe der Korbblütler.

Geeignete Süßgräser sind beispielsweise Liebesgräser (Eragrostis). Süßgräser werden erfindungsgemäß insbesondere in Form zerkleinerter Körner und vorzugsweise als Mehl verwendet. Dabei sind Süßgräser bevorzugt, deren Körner bezogen auf die Trockenmasse mindestens 5 Gew.-% und insbesondere mindestens 8 Gew.-% Proteine enthalten und dabei insbesondere einen Tryptophan-Anteil von mindestens 0,5 g/100 g Protein und insbesondere mindestens 1,0 g/100 g Protein aufweisen. Vorzugsweise enthalten die Körner bezogen auf die Trockenmasse weniger als 5 Gew.-% und insbesondere weniger als 2,5 Gew.-% Fett und/oder mindestens 50 Gew.-% und insbesondere mindestens 55 Gew.-% Kohlenhydrate. Insbesondere sind solche Süßgräser bevorzugt, deren Körner bezogen auf die Trockenmasse mindestens 20 Gew.-% und am meisten bevorzugt mindestens 25 Gew.-% prebiotische Kohlenhydrate enthalten. Ein besonders bevorzugtes Süßgras ist Teff, insbesondere in Form von Teffmehl. Typischerweise enthält die Getränkezusammensetzung 0,1 bis 20 Gew.-ö, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% zerkleinerte Pflanzenteile mindestens einer Pflanze aus der Gruppe der Süßgräser.

Es hat sich als vorteilhaft erwiesen, wenn die Getränkezusammensetzung einen geringen glykämischen Index von weniger als 70, insbesondere weniger als 50 und besonders bevorzugt weniger als 30 aufweist. Daher sind insbesondere solche Pflanzenbestandteile bevorzugt, die einen geringen Anteil, wie beispielsweise weniger als 20 Gew.-%, insbesondere weniger als 10 Gew.-% und besonders bevorzugt weniger als 7,5 Gew.-%, bezogen auf die Trockenmasse, an Kohlenhydraten oder einen hohen Anteil, wie beispielsweise mindestens 20 Gew.-%, insbesondere mindestens 40 Gew.-% und besonders mindestens 50 Gew.-%, bezogen auf die Trockenmasse, an prebiotischen Kohlenhydraten aufweisen.

Besonders bevorzugt enthält die Getränkezusammensetzung die oben definierten Bestandteile (a), (b) und (c) in einem Gewichtsverhältnis von 10:10:1 bis 0,1:0,1:1, insbesondere 8:4:1 bis 0,5:0,25:1, bevorzugt 4:2:1 bis 1:0,5:1 und am meisten bevorzugt 3:1,25:1 bis 1,5:0,75:1.

In bevorzugten Ausführungsformen enthält die Getränkezusammensetzung eine Kombination von Lupinenmehl und Topinamburknollenmehl, eine Kombination von Lupinenmehl und Teffmehl und/oder eine Kombination von Topinamburknollenmehl und Teffmehl. Eine Getränkezusammensetzung, die eine Kombination von Lupinenmehl, Topinamburknollenmehl und Teffmehl enthält, ist besonders bevorzugt.

Eine erfindungsgemäße Getränkezusammensetzung enthält neben zerkleinerten Pflanzenteilen eine geeignete Menge an Flüssigkeit, insbesondere Wasser und/oder eine Getränkekomponente pflanzlichen order tierischen Ursprungs. Üblicherweise liegen die zerkleinerten Pflanzenteile in der erfindungsgemäßen Getränkezusammensetzung in einer Form vor, in der zumindest ein Teil der zerkleinerten Pflanzenteile zusätzliches Wasser physikalisch und/oder chemisch gebunden hat. Bevorzugt liegen die zerkleinerten Pflanzenteile in der Zusammensetzung in mindestens teilweise gequollener Form vor. In einer bevorzugten Ausführungsform ist die erfindungsgemäße Getränkezusammensetzung dadurch erhältlich, dass die zerkleinerten Pflanzenteile, insbesondere Pflanzenmehle, mit Fruchtsaft, Gemüsesaft, Milch und/oder Wasser gemischt und über einen Zeitraum von mindestens 1 Stunde, insbesondere mindestens 12 Stunden, beispielsweise mindestens 24 Stunden, mindestens 48 Stunden und am meisten bevorzugt mindestens 96 Stunden in Kontakt gehalten werden. Es hat sich überraschend gezeigt, dass die wirksamen Bestandteile der Pflanzenteile durch die Formulierung als Getränk nach der Einnahme besonders vorteilhaft verfügbar sind.

Vorzugsweise enthält die Getränkezusammensetzung Fruchtsaft, Gemüsesaft und/oder Milch.

Der Begriff "Fruchtsaft" bezeichnet hier allgemein ein aus einer Frucht gewonnenes flüssiges Erzeugnis und umfasst insbesondere auch Direktsaft, Fruchtsaftgetränk, Fruchtnektar, Fruchtmark und Fruchtsaftkonzentrat sowie deren Mischungen mit Wasser. Beispiele für Fruchtsaft sind Apfelsaft, Passionsfruchtsaft, Traubensaft und Bananensaft.

Der Begriff "Gemüsesaft" bezeichnet hier allgemein ein aus einem Gemüse gewonnenes flüssiges Erzeugnis und umfasst insbesondere auch Gemüsesaftkonzentrat und Gemüsemark sowie deren Mischungen mit Wasser. Beispiele für geeignete Gemüse sind Sellerie, Schwarzkarotte, Karotte, Kürbis, Tomate und Rote Beete.

Insbesondere können erfindungsgemäß Mischungen von Fruchtsaftkonzentrat, Fruchtmark, Gemüsesaftkonzentrat oder Gemüsemark mit einer geeigneten Menge Wasser verwendet werden. Saftkonzentrate werden üblicherweise mindestens mit der zur Rekonstitution des entsprechenden Safts erforderlichen Menge Wasser eingesetzt. Besonders bevorzugt ist eine Getränkezusammensetzung, die 1 bis 70 Gew.-%, insbesondere 5 bis 60 Gew.-%, bevorzugt 10 bis 50 Gew.-% und besonders bevorzugt 20 bis 40 Gew.-% Fruchtsaftkonzentrat, Fruchtmark, Gemüsesaftkonzentrat und/oder Gemüsemark enthält.

Es können auch mehrere Früchte und/oder Gemüse in Kombination verwendet werden. Insbesondere kann die Getränkezusammensetzung Fruchtsaft von mindestens zwei Früchten und bevorzugt von mindestens drei Früchten enthalten. Besonders bevorzugt sind Kombinationen von Apfelsaft, Bananensaft und Passionsfruchtsaft und Kombinationen von Traubensaft und Passionsfruchtsaft.

Der Begriff "Milch" bezeichnet hier allgemein ein eiweißhaltiges Getränk tierischen oder pflanzlichen Ursprungs. Beispiele für Milch tierischen Ursprungs sind Kuhmilch, insbesondere Rohmilch, Vollmilch, fettarme Milch und Magermilch. Beispiele für Milch pflanzlichen Ursprungs sind Sojamilch, Reismilch, Hafermilch und Mandelmilch.

Besonders bevorzugt ist eine Getränkezusammensetzung, die 50 bis 99 Gew.-%, insbesondere 70 bis 98 Gew.-%, bevorzugt 80 bis 97 Gew.-%, besonders bevorzugt 90 bis 96 Gew.-% und am meisten bevorzugt 90 bis 95 Gew.-% Fruchtsaft, Gemüsesaft und/oder Milch enthält.

Typischerweise enthält die erfindungsgemäße Getränkezusammensetzung 25 bis 99 Gew.-%, insbesondere 35 bis 95 Gew.-%, bevorzugt 45 bis 85 Gew.-% und besonders bevorzugt 55 bis 75 Gew.-% Wasser.

In einer bevorzugten Ausführungsform enthält die erfindungsgemäße Getränkezusammensetzung
(a) 0,1 bis 20 Gew.-%, insbesondere 0,5 bis 10 Gew.-%, bevorzugt 1 bis 5 Gew.-%, besonders bevorzugt 2 bis 4 Gew.-% und am meisten bevorzugt 2,5 bis 3,5 Gew.-% Lupinenmehl;
(b) 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% Topinamburknollenmehl;
(c) 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% Teffmehl; und
(d) 50 bis 99 Gew.-%, insbesondere 70 bis 98 Gew.-%, bevorzugt 80 bis 97 Gew.-%, besonders bevorzugt 90 bis 96 Gew.-% und am meisten bevorzugt 90 bis 95 Gew.-% Fruchtsaft, Gemüsesaft und/oder Milch.

In einer besonders bevorzugten Ausführungsform enthält die erfindungsgemäße Getränkezusammensetzung
(a) 0,1 bis 20 Gew.-%, insbesondere 0,5 bis 10 Gew.-%, bevorzugt 1 bis 5 Gew.-%, besonders bevorzugt 2 bis 4 Gew.-% und am meisten bevorzugt 2,5 bis 3,5 Gew.-% Lupinenmehl;
(b) 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% Topinamburknollenmehl;
(c) 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% Teffmehl;
(d) 1 bis 70 Gew.-%, insbesondere 5 bis 60 Gew.-%, bevorzugt 10 bis 50 Gew.-% und besonders bevorzugt 20 bis 40 Gew.-% Fruchtsaftkonzentrat und/oder Fruchtmark, insbesondere Apfelsaftkonzentrat, Bananenmark, Traubensaftkonzentrat und/oder Passionsfruchtkonzentrat; und
(e) 25 bis 99 Gew.-%, insbesondere 35 bis 95 Gew.-%, bevorzugt 45 bis 85 Gew.-% und besonders bevorzugt 55 bis 75 Gew.-% Wasser.

Die erfindungsgemäße Zusammensetzung ist vorzugsweise im Wesentlichen und am meisten bevorzugt völlig frei von Zusatzstoffen wie künstlichen Aromen, Emulgatoren, Füll- und Bindemitteln, Geschmacksverstärkern und Konservierungsmitteln. Es ist weiterhin bevorzugt, dass die Zusammensetzung (außer Wasser) ausschließlich naturbelassene Inhaltsstoffe aus biologischer Erzeugung enthält.

Die erfindungsgemäße Getränkezusammensetzung weist üblicherweise eine dynamische Viskosität bei 20 °C im Bereich von 50 bis 300 mPas und vorzugsweise im Bereich von 100 bis 200 mPas auf. Die Viskosität kann insbesondere mittels eines Brookfield LVF-Viskosimeters mit entsprechender Spindel bestimmt werden. Die Größe der in der Getränkezusammensetzung enthaltenen stückigen Einlagen liegt typischerweise im Bereich von 100 bis 200 mesh.

In einem zweiten Aspekt betrifft die Erfindung ein Verfahren zur Herstellung der erfindungsgemäßen Getränkezusammensetzung gemäß dem ersten Aspekt der Erfindung, bei dem zerkleinerte Pflanzenteile mit Fruchtsaft, Gemüsesaft, Milch und/oder Wasser gemischt und über einen Zeitraum von mindestens 1 Stunde, insbesondere mindestens 12 Stunden, beispielsweise mindestens 24 Stunden, mindestens 48 Stunden und am meisten bevorzugt mindestens 96 Stunden in Kontakt gehalten werden.

Die zerkleinerten Pflanzenteile können nach an sich bekannten Methoden erhalten werden, beispielsweise mittels eines üblichen Mahlverfahrens. Vorzugsweise werden die Pflanzenteile naturbelassen verwendet, d.h. abgesehen von der Entfernung nichtessbarer Teile wie Spelzen und Hülsen und der mechanischen Zerkleinerung wird keine Veränderung der Pflanzenteile vorgenommen. Insbesondere werden die zerkleinerten Pflanzenteile vorzugsweise weder ganz noch teilweise entfettet.

Die Herstellung der erfindungsgemäßen Getränkezusammensetzung erfolgt vorzugsweise ohne Verwendung von Zusatzstoffen wie freien Aminosäuren, Vitaminen, Kohlenhydraten (insbesondere Zucker), Proteinen oder Fetten sowie künstlichen Aromen, Emulgatoren, Füll- und Bindemitteln, Geschmacksverstärkern und Konservierungsmitteln. Besonders bevorzugt werden zur Herstellung der Getränkezusammensetzung (außer Wasser) ausschließlich naturbelassene Inhaltsstoffe aus biologischer Erzeugung verwendet.

Nach dem Vermischen der zerkleinerten Pflanzenteile mit Fruchtsaft, Gemüsesaft, Milch und/oder Wasser kann die erhaltene Mischung beispielsweise in Glas- oder Kunststoffflaschen abgefüllt werden. Das oben beschriebene in Kontakt halten kann dabei zum überwiegenden Teil nach dem Abfüllen erfolgen. Die Abfüllung kann nach an sich bekannten Verfahren beispielsweise als Kaltabfüllung, Heißabfüllung, Pasteurisierung, Sterilabfüllung oder aseptische Abfüllung durchgeführt werden.

In einem dritten Aspekt betrifft die Erfindung eine Getränkezusammensetzung, die nach dem Verfahren gemäß dem zweiten Aspekt der Erfindung erhältlich ist.

In einem weiteren Aspekt betrifft die Erfindung die Getränkezusammensetzung gemäß dem ersten oder dritten Aspekt der Erfindung zur Anwendung bei der Behandlung einer Störung des Serotoninstoffwechsels. Serotonin hat im Zentralnervensystem unter anderem wesentlichen Einfluss auf Stimmung und Verhalten sowie Wahrnehmung, Sensorik, Schlaf, Temperaturregulation, Schmerzempfindung und -Verarbeitung, Appetit, Sexualverhalten und Hormonsekretion. Insbesondere eignet sich die erfindungsgemäße Getränkezusammensetzung zur Anwendung bei der Behandlung eines Zustands oder einer Krankheit ausgewählt aus der Gruppe bestehend aus Schlafstörungen, Essstörungen, affektiven Störungen, Depressionen, Angststörungen, Zwangs störungen und chronischen Schmerzen.

Dabei wird die Getränkezusammensetzung üblicherweise einmal, zweimal oder dreimal täglich, vorzugsweise einmal täglich, und insbesondere in einer Tagesdosis von 125 bis 150 g eingenommen. Vorzugsweise wird die Zusammensetzung morgens auf nüchternen Magen und insbesondere mindestens 10 bis 60 Minuten, bevorzugt mindestens 30 Minuten, vor der ersten Mahlzeit eingenommen. Bei der Behandlung von Schlafstörungen oder Depressionen wird die Zusammensetzung abends 10 bis 60 Minuten, bevorzugt 20 bis 30 Minuten, vor dem Schlafengehen eingenommen.

Die erfindungsgemäße Getränkezusammensetzung eignet sich auch dazu, eine nicht medizinisch erforderliche Erhöhung des Serotoninspiegels im Gehirn zu bewirken. In einem weiteren Aspekt betrifft die Erfindung daher die Verwendung der Getränkezusammensetzung gemäß dem ersten oder dritten Aspekt der Erfindung zur Anregung des Serotoninstoffwechsels im Gehirn, typischerweise bei Personen, die nicht an einer Störung des Serotoninstoffwechsels leiden, und insbesondere zur Verbesserung des Allgemeinbefindens, der Leistungsfähigkeit, der Konzentration, der Fähigkeit zur Entspannung und vor allem der mentalen Ausgeglichenheit. Bevorzugte Ausführungsformen bezüglich der Dosierung und Einnahme der Zusammensetzung sind wie oben definiert.

Die Erfindung wird nachfolgend anhand von Beispielen näher erläutert.

### Beispiele

### Beispiel 1

Getränkezusammensetzung mit 3 Saft-Komponenten (Zusammensetzung für 1 Liter)

Es wurde eine Getränkezusammensetzung mit den folgenden Komponenten hergestellt:

| **Komponente** | **Menge (g)** |
|---|---|
| Apfelsaftkonzentrat | 104 |
| Bananenpüree | 235 |
| Passionsfruchtsaftkonzentrat | 28 |
| Lupinenmehl | 32 |
| Topinamburknollenmehl | 16 |
| Teffmehl | 16 |
| Wasser | 624 |
| **Total** | **1055** |

Die Pflanzenmehle wurden mit den Saftkonzentraten, Fruchtpüree und Wasser gemischt. Anschließend wurde die Mischung luftdicht in Glas- oder Kunststoffflaschen abgefüllt und vor der Verwendung mindestens 96 Stunden stehengelassen. Nach dem Öffnen war die Getränkezusammensetzung bei 2 bis 8 °C mindestens 3 bis 6 Tage haltbar.

### Beispiel 2

Getränkezusammensetzung mit 2 Saft-Komponenten

### (Zusammensetzung für 1 Liter)

Analog zu dem für Beispiel 1 beschriebenen Verfahren wurde eine Getränkezusammensetzung mit den folgenden Komponenten hergestellt:

| **Komponente** | **Menge (g)** |
|---|---|
| Traubensaftkonzentrat | 186 |
| Passionsfruchtsaftkonzentrat | 52 |
| Lupinenmehl | 32 |
| Topinamburknollenmehl | 16 |
| Teffmehl | 16 |
| Wasser | 763 |
| **Total** | **1055** |

### Beispiel 3

### Anwendungstest

Die Testgruppe bestand aus 24 gesunden Erwachsenen im Alter von 25 bis 54 Jahren.

Vor Beginn der eigentlichen Testphase wurde für jeden Probanden eine umfassende Anamnese einschließlich der Erfassung des medizinischen Status (Allgemeinbefinden, Schlafverhalten, psychischer Status, Stuhlgang, Allergien, Medikamenten- und Drogenkonsum, Impfstatus, Größe, Gewicht und Blutdruck) und der Nährstoffversorgung (Ess- und Trinkgewohnheiten sowie dadurch eventuell bedingte Mangelversorgung an Vitalstoffen) erhoben. Anschließend nahmen die Probanden über einen Zeitraum von 21 Tagen jeweils morgens auf nüchternen Magen 125 ml der Getränkezusammensetzung gemäß Beispiel 1 zu sich. Nach der Einnahme der Getränkezusammensetzung mussten die Probanden jeweils 30 Minuten warten, bevor sie feste Nahrung zu sich nehmen durften. Im Laufe der Testphase brachen 2 Personen den Test aus persönlichen Gründen ab.

Für die verbleibenden 22 Probanden wurde nach Abschluss der Testphase die oben beschriebene Anamnese wiederholt. Durch individuellen Vergleich mit dem Status vor der Testphase wurden bei allen Probanden die folgenden Veränderungen festgestellt:
- verbessertes Einschlaf- und Durchschlafverhalten
- deutliche Stimmungsaufhellung (Ausgeglichenheit, Geduldigkeit, bessere Laune)
- erhöhte Stuhlfrequenz und verbesserte Stuhlregulation
- vermindertes Verlangen nach Alkohol und Süßigkeiten
- verminderte PMS- Symptome (bei den weiblichen Probanden)
- gesteigerte Leistungsfähigkeit und Lust auf körperliche Bewegung, erhöhte Vitalität
- verbesserte Konzentration und mentale Stärkung

## Patentansprüche

1. Gesundheitsfördernde Getränkezusammensetzung, die zerkleinerte Pflanzenteile von Pflanzen ausgewählt aus mindestens zwei der Gruppen Hülsenfrüchtler, Korbblütler und Süßgräser enthält.

2. Getränkezusammensetzung nach Anspruch 1 enthaltend
(a) zerkleinerte Samen mindestens einer Pflanze aus der Gruppe der Hülsenfrüchtler;
(b) zerkleinerte Knollen mindestens einer Pflanze aus der Gruppe der Korbblütler; und
(c) zerkleinerte Körner mindestens einer Pflanze aus der Gruppe der Süßgräser.

3. Getränkezusammensetzung nach Anspruch 1 oder 2, bei der der Hülsenfrüchtler Lupine, und insbesondere Süßlupine, ist.

4. Getränkezusammensetzung nach einem der Ansprüche 1 bis 3, die 0,1 bis 20 Gew.-%, insbesondere 0,5 bis 10 Gew.-%, bevorzugt 1 bis 5 Gew.-%, besonders bevorzugt 2 bis 4 Gew.-% und am meisten bevorzugt 2,5 bis 3,5 Gew.-% zerkleinerte Pflanzenteile mindestens einer Pflanze aus der Gruppe der Hülsenfrüchtler enthält.

5. Getränkezusammensetzung nach einem der Ansprüche 1 bis 4, bei der der Korbblütler Topinambur ist.

6. Getränkezusammensetzung nach einem der Ansprüche 1 bis 5, die 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% zerkleinerte Pflanzenteile mindestens einer Pflanze aus der Gruppe der Korbblütler enthält.

7. Getränkezusammensetzung nach einem der Ansprüche 1 bis 6, bei der das Süßgras Teff ist.

8. Getränkezusammensetzung nach einem der Ansprüche 1 bis 7, die 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% zerkleinerte Pflanzenteile mindestens einer Pflanze aus der Gruppe der Süßgräser enthält.

9. Getränkezusammensetzung nach einem der Ansprüche 2 bis 8, die die Bestandteile (a), (b) und (c) in einem Gewichtsverhältnis von 10:10:1 bis 0,1:0,1:1, insbesondere 8:4:1 bis 0,5:0,25:1, bevorzugt 4:2:1 bis 1:0,5:1 und am meisten bevorzugt 3:1,25:1 bis 1,5:0,75:1 enthält.

10. Getränkezusammensetzung nach einem der Ansprüche 1 bis 9, die Lupinenmehl, Topinamburknollenmehl und Teffmehl enthält.

11. Getränkezusammensetzung nach einem der Ansprüche 1 bis 10, die Fruchtsaft, Gemüsesaft und/oder Milch enthält.

12. Getränkezusammensetzung nach einem der Ansprüche 1 bis 11, die
(a) 0,1 bis 20 Gew.-%, insbesondere 0,5 bis 10 Gew.-%, bevorzugt 1 bis 5 Gew.-%, besonders bevorzugt 2 bis 4 Gew.-% und am meisten bevorzugt 2,5 bis 3,5 Gew.-% Lupinenmehl;
(b) 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% Topinamburknollenmehl;
(c) 0,1 bis 20 Gew.-%, insbesondere 0,25 bis 5 Gew.-%, bevorzugt 0,5 bis 2,5 Gew.-%, besonders bevorzugt 1 bis 2 Gew.-% und am meisten bevorzugt 1,25 bis 1,75 Gew.-% Teffmehl; und
(d) 50 bis 99 Gew.-%, insbesondere 70 bis 98 Gew.-%, bevorzugt 80 bis 97 Gew.-%, besonders bevorzugt 90 bis 96 Gew.-% und am meisten bevorzugt 90 bis 95 Gew.-% Fruchtsaft, Gemüsesaft und/oder Milch enthält.

13. Getränkezusammensetzung nach einem der Ansprüche 1 bis 12 erhältlich nach einem Verfahren, bei dem zerkleinerte Pflanzenteile mit Fruchtsaft, Gemüsesaft, Milch und/oder Wasser gemischt und über einen Zeitraum von mindestens 1 Stunde, insbesondere mindestens 12 Stunden, beispielsweise mindestens 24 Stunden, mindestens 48 Stunden und am meisten bevorzugt mindestens 96 Stunden in Kontakt gehalten werden.

14. Getränkezusammensetzung nach einem der Ansprüche 1 bis 13 zur Anwendung bei der Behandlung einer Störung des Serotoninstoffwechsels.

15. Verwendung einer Getränkezusammensetzung gemäß einem der Ansprüche 1 bis 13 zur Anregung des Serotoninstoffwechsels.
